# EUROPEAN PATENT APPLICATION

(11) **EP 2 567 735 A1**
(43) Date of publication of application: **13.03.2013**
(21) Application number: 10851030.6
(22) Date of filing: 23.11.2010
(51) Int. Cl.: A62B 23/06, A61F 5/56

(54) **DEVICE FOR THE SYMPTOMATIC RELIEF OF NASAL ALLERGIES**

(30) Priority: 07.05.2010 ES 201030439 U
(71) Applicant: Orts Paya, David, 03430 Alicante (ES); Ribera Juan, José Antonio, 03430 Alicante (ES); Rico Sanchis, Francisco José, 03430 Alicante (ES); Berenguer Rico, José Pascual, 03430 Alicante (ES); Albero Mira, Cesar, 03430 Alicante (ES); Moles Moles, José, 03430 Alicante (ES)
(72) Inventor: Orts Paya, David, 03430 Alicante (ES); Ribera Juan, José Antonio, 03430 Alicante (ES); Rico Sanchis, Francisco José, 03430 Alicante (ES); Berenguer Rico, José Pascual, 03430 Alicante (ES); Albero Mira, Cesar, 03430 Alicante (ES); Moles Moles, José, 03430 Alicante (ES)
(74) Representative: Isern-Jara, Nuria
(86) International application number: PCT/ES2010/070764
(87) International publication number: WO 2011/138474

(57) **Abstract**

The invention relates to a device for the symptomatic relief of nasal allergies, comprising a single one-piece body (I) formed in turn by two tubular elements (2) designed to be inserted into the nostrils and to maintain the nostrils open. The tubular elements have a plurality of external annular rounded projections (3) and the tubular elements (2) are linked to one another by an intermediate bridge (4).

## Description

### TECHNICAL FIELD OF THE INVENTION

The invention herein relates to the technical field of devices for the symptomatic relief of those allergies produced by elements entering via the nostrils, namely for the symptomatic relief of allergies in cases where there are obstructions or congenital malformations or of any kind in the nostrils, and more specifically to devices with tubular elements that are inserted into the nostrils to ease breathing and to relieve allergy symptoms.

### BACKGROUND OF THE INVENTION

Currently, many means are known that enable better breathing, in certain cases where the user has mucus, malformations in the nose etc., irrespective of medical treatments.

These means may consist of a simple strip-like element attached externally on the bridge of the nose, such that the element exerts traction on the sides of the nose and opens the nostrils. This device has been used by athletes and other persons and results in respect of opening the nostrils have not been ideal.

On the other hand, another device is known that is formed by two short tubes inserted into the nostrils, linked by a bridge which acts as a joining element between said tubes and as a stop element to limit the maximum depth it can penetrate into the nostrils. These short tubes are provided with a screw thread on the inside for coupling corresponding end pieces that form the entrance to the air inlet from the nostrils to the outside. The drawback of this type of device is that it comprises three separate elements or pieces, two for the short tubes and another for the bridge. The end pieces that can be coupled to the threaded parts of the tubes must also be added, making it a device formed by five pieces. On the one hand, this represents a high cost of manufacturing of the device and a greater need for handling the various parts when assembling the device.

There are high costs associated with the above, as well as the inconvenience caused to users. Additionally, on removing the device the end pieces could remain lodged inside the nostrils if these have been incorrectly threaded, causing discomfort and further problems for the user.

Another drawback with this type of device is that inserting it into the nostrils prevents the hairs inside said nostrils from performing their function, which is to prevent small particles such as dust, sand, plant seeds and pollutants from entering the nostrils. All air pathogens, listed in the previous sentence, enter the lungs directly without being filtered. Therefore, using these devices could cause additional discomfort to the user, including an increase in the undesirable effects of allergies.

However, no device is known that performs the above functions and leads to improved health for individuals with allergies, relieving the usual inconveniences associated with this type of medical condition.

It was therefore desirable to come up with a device that would efficiently open the nostrils avoiding the drawbacks existing in previous devices of the state of the art and that would improve the health of the users who suffer from allergies.

### DESCRIPTION OF THE INVENTION

This invention solves the problems in the prior art by means of a device for the symptomatic relief of nasal allergy, comprising a single one-piece body, formed in turn by two short tubular elements designed to be inserted into the nostrils and to maintain the nostrils open. The tubular elements are linked to one another by an intermediate bridge which acts as a stop elementto prevent excessive penetration of the tubular elements into the nostrils.

This single piece provides a number of advantages over existing devices in the prior art, such as greater structural simplicity and fewer elements, which results in simpler manufacturing, no need for parts to be coupled and no end pieces that could remain inside the nostrils as a result of being incorrectly secured.

Additionally, the tubular elements have a plurality of external annular rounded projections arranged on their outer surface making it easier for them to be inserted into the nostrils, thereby preventing scratches or ulcerations inside the nostrils.

According to a preferred embodiment of the invention, the tubular elements have helically-arranged fins disposed along said tubular elements, which make it more difficult for certain particles, such as dust, sand or plant seeds to enter the nostrils, thereby alleviating and reducing allergy symptoms. As such, these fins act as a substitute to the hairs inside the nostrils that cannot fulfil their protective function when the tubular elements are inserted. Additionally, since the fins are going to be wet from being in the nasal passage, any particles that might enter the nose would come up against another obstacle in order to reach the inner respiratory system.

### DESCRIPTION OF THE FIGURES

For a better understanding of the invention, an embodiment of the invention referring to a number of figures, illustrated by way of non-limiting example, is described herein below.

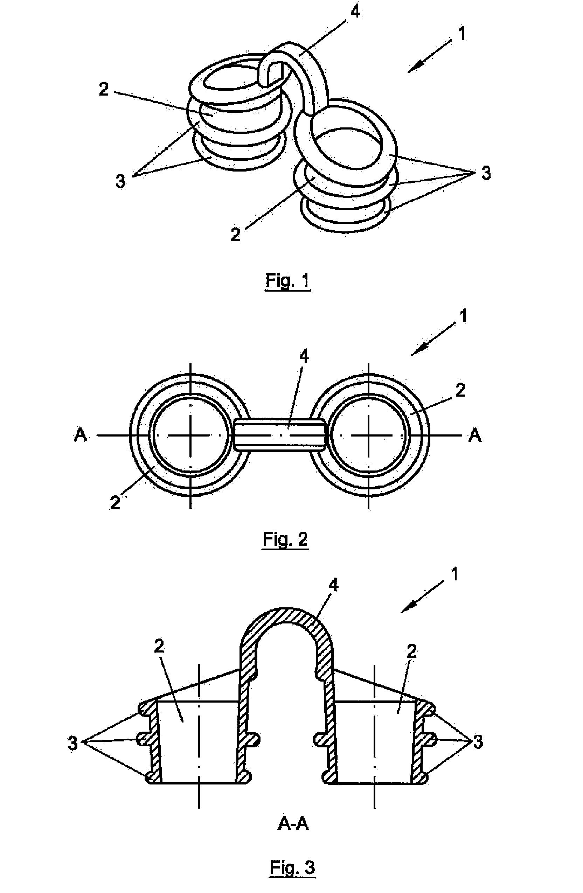

Figure 1 shows a representation according to a general perspective of the device for opening the nostrils, object of the invention herein.
Figure 2 is a front elevation view of the device in figure 1.
Figure 3 is a sectional view of the AA plane in figure 2.
Figure 4 shows a particular embodiment of the device for opening the nostrils with a continuous fin arranged helically inside each of the tubular elements.
Figure 5 is a front elevation view of the device in figure 4.
Figure 6 is an alternative embodiment of the device with separate fins in layers, arranged helically inside the tubular elements.
Figure 7 is a front elevation view of the device in figure 6.

These figures relate to a set of elements which include:
1. a one-piece body
2. tubular elements
3. external projections of the tubular elements
4. an intermediate bridge
5. inner fins of the tubular elements

### DESCRIPTION OF PREFERRED EMBODIMENTS OF THE INVENTION

As can be seen in the figures, the object of this invention is a device for the symptomatic relief of allergies comprising a single one-piece body 1, which is preferably made from a flexible material and formed in turn by two tubular elements 2 intended to be inserted into the nostrils of a user and to maintain the nostrils open. As can be seen in the figures, the tubular elements 2 have a plurality of external annular rounded projections 3 making it easier for them to be inserted into the nostrils, thereby preventing scratches or ulcerations inside the nostrils.

The tubular elements 2 are linked to one another by an intermediate bridge 4, which acts as a buffer preventing excessive penetration of the tubular elements 2 inside the nostrils.

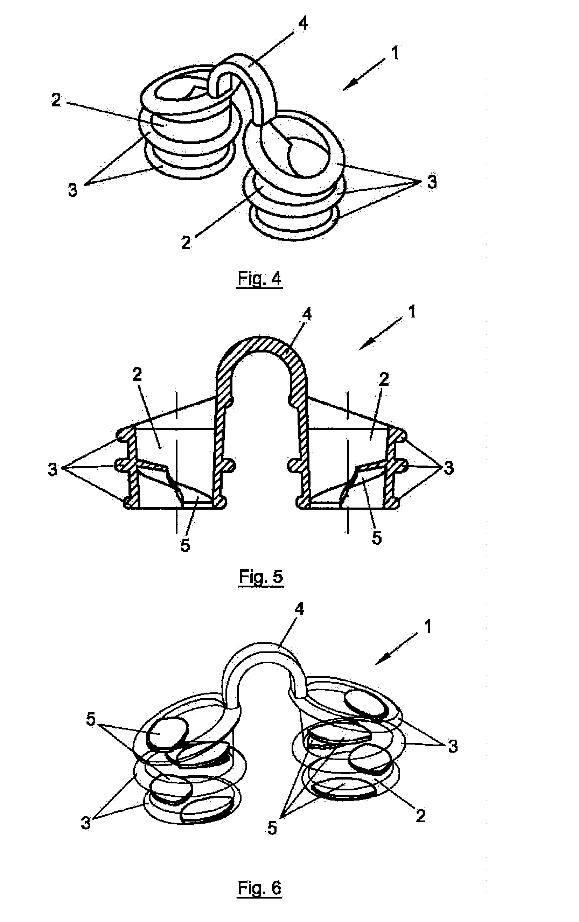

Figures 4 and 5 show a particular embodiment of the invention in which each tubular element 2 has a continuous fin 5 inside, arranged helically along the tubular element 2, which makes it difficult for particles such as dust, sand, or plant seeds to enter the nostrils, which helps reduce the effects of allergies.

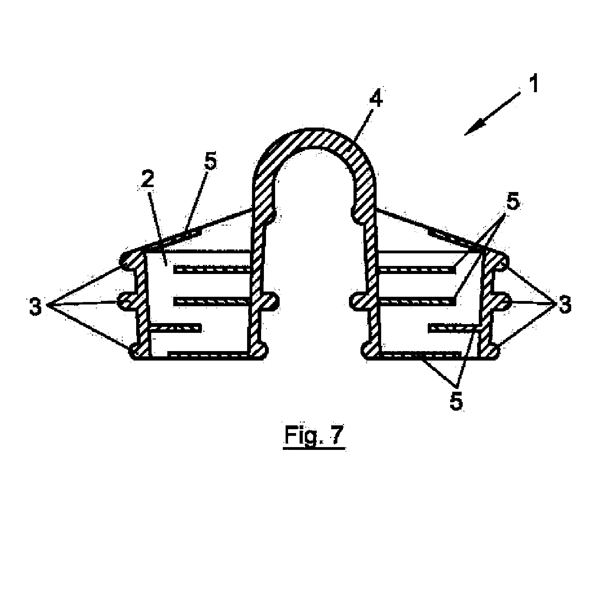

Figures 6 and 7 show an alternative embodiment to the above, in which each tubular element 2 has a plurality of short fins 5 inside, disposed helically along the tubular element 2, achieving the same effect as the previous embodiment.

The fins 5 are made preferably from medical-grade silicone, although they may be made from another flexible material.

Having described the invention clearly, it is noted that the particular embodiments described above may be amended in detail provided the fundamental principle and the essence of the invention is not altered.

## Claims

1. A device for the symptomatic relief of nasal allergies, **characterised in that** it comprises a one-piece body (1), comprising in turn:
- two tubular elements (2) intended to be inserted into the nostrils and to maintain the nostrils open, which comprise a plurality of external annular rounded projections (3) on its outer surface, said tubular elements (2) being linked to one another by
- an intermediate bridge (4).

2. A device for the symptomatic relief of nasal allergies, according to the claim 1, **characterised in that** the tubular elements (2) comprise fins (5) in their interior, arranged helically along said tubular elements (2).

3. A device for the symptomatic relief of nasal allergies, according to the previous claim, **characterised in that** the fins (5) are made from medical-grade silicone.

4. A device for the symptomatic relief of nasal allergies, according to any of the preceding claims, **characterised in that** the one-piece body (1) is made from a flexible material.
